# EUROPEAN PATENT APPLICATION

(11) **EP 3 677 544 A1**
(43) Date of publication of application: **08.07.2020**
(21) Application number: 18852465.6
(22) Date of filing: 30.03.2018
(51) Int. Cl.: C01B 32/336, C01B 32/318

(54) **HIGH-PERFORMANCE SPHERICAL ACTIVATED CARBON, PREPARATION METHOD THEREFOR AND USE THEREOF**

(30) Priority: 01.09.2017 CN 201710781322
(71) Applicant: Shenzhen Global Greenland New Materials Co., Ltd., Shenzhen, Guangdong 518100 (CN)
(72) Inventor: CHANG, Mingzhu, Shenzhen Guangdong 518100 (CN)
(74) Representative: Gille Hrabal
(86) International application number: PCT/CN2018/081431
(87) International publication number: WO 2019/041807

(57) **Abstract**

The present disclosure provides a high-performance spherical activated carbon, preparation method and use thereof. The activated carbon achieves excellent physical or mechanical properties at a low specific surface area. Moreover, by using the preparation method according to the disclosure, the spherical activated carbon with large particle size can be prepared in good yield with low cost.

## Description

This application claims the benefit of and priority to the prior Chinese patent application No. 201710781322.8, entitled "HIGH-PERFORMANCE SPHERICAL ACTIVATED CARBON, PREPARATION METHOD AND USE THEREOF" and filed before the Chinese National Intellectual Property Office on September 1, 2017, which is hereby incorporated by reference in its entirety.

### TECHNICAL FIELD

The present disclosure belongs to the technical field of adsorbent material, and in particular relates to high-performance spherical activated carbon, preparation method and application thereof.

### BACKGROUND

Activated carbon has fairly unspecific adsorptive properties and therefore is the most widely used adsorbent. Activated carbon is generally obtained by carbonization and subsequent activation of a carbonaceous raw material. Such raw material is preferably selected from those that can produce an economically reasonable yield, because the weight losses brought by the removal of volatile components during carbonization and by the subsequent burn-out during activation are considerable.

The properties, such as porosity, hardness or brittleness, of the activated carbon product can vary depending on the raw material. Since conventional starting materials can be widely selected from coconut shell, charcoal, wood, peat, stone coal and asphalt, etc., their application value can be improved in the activated carbon. However, depending on different processes, activated carbon can be used in various forms, such as powder carbon, flake carbon, granular carbon, molded carbon and also spherical activated carbon used since the end of the 1970s.

Spherical activated carbon has a number of advantages over other forms of activated carbon such as powder carbon, flake carbon, granular carbon, molded carbon and the like. These advantages comprise free flowing, abrasion resistant or more precisely dustless and hard that make the spherical activated carbon useful or even indispensable for certain applications. In particular, spherical activated carbon is in great demand for particular applications, for example, because of its specific form and high abrasion resistance.

So far spherical activated carbon is still mostly being produced by multistage, very costly and inconvenient processes. The best-known process consists in producing spherules from bituminous coal tar pitch and suitable asphaltic residues from the petrochemical industry, which are oxidized to render them unmeltable and then smoldered and activated. For example, spherical activated carbon can also be produced in a multistage process proceeding from asphalt. However, these multistage processes are very cost intensive and the associated high cost of this spherical activated carbon prevents many applications wherein spherical activated carbon ought to be preferable by virtue of its properties.

There are also many processes for producing spherical activated carbon. For example, a carbon-containing raw material can be subjected to carbonization and activation treatments, by regulating and controlling the processing parameters of each stage, to achieve the preparation of spherical activated carbon. However, there are still many defects in the preparation of spherical activated carbon by the existing process. For example, it is difficult to obtain spherical activated carbon with larger particle size and satisfied mechanical and strength properties in existing practical production processes, so that their practical application scope are greatly limited. Also, it is difficult for the existing processes to realize the coordination between the particle size, strength, pore diameter, specific surface area and adsorption performance of spherical activated carbon. Therefore, it is necessary to provide a spherical activated carbon, which has better mechanical properties than the prior art when it has a particular specific surface area. In addition, the spherical activated carbon can possess a larger particle size as much as possible on the premise of avoiding its performance degradation. Furthermore, it is necessary to develop a more stable and suitable preparation method of such spherical activated carbon for large-scale production.

### SUMMARY

In order to improve the above problems existing in the prior art, the present disclosure provides a spherical activated carbon, having a specific surface area B less than 1250 m²/ g.

For example, 600 m²/g ≤ B ≤ 1200 m²/g. As an exemplary example, 700 m²/g ≤ B ≤ 1100 m²/g.

According to the present disclosure, median particle size D₅₀ of the spherical activated carbon can be 0.2-1.5mm, such as 0.5-1.3 mm or 0.7-1.2 mm. As an exemplary example, the D₅₀ can be 0.5 mm, 0.6 mm, 0.7 mm, 0.8 mm, 0.9 mm, 1.0 mm, 1.1 mm or 1.2 mm.

Preferably, the median pore size of the spherical activated carbon is 1∼4 nm, such as 1.5∼3.8 nm or 1.8∼3.8 nm.

According to the present disclosure, the compressive strength of the spherical activated carbon can be 10∼300 N. Such as 40-150 N, 50-140N or 50-130 N.

According to the present disclosure, the term compressive strength refers to the maximum pressure that a spherical activated carbon can bear.

According to the present disclosure, the cracking rate of the spherical activated carbon can be less than 10.0%, such as 1.0∼10.0% or 1.5∼6.0%, preferably less than 5.0%, such as 3.0∼5.0%.

According to the present disclosure, the bulk density of the spherical activated carbon can be 300-1000 g/L, preferably 400-800 g/L, such as 450-700 g/L.

According to the present disclosure, the iodine adsorption value of the spherical activated carbon is 400-1100mg/g, preferably 500-1000 mg/g, such as 600-950 mg/g.

According to the present disclosure, the raw material for preparing the spherical activated carbon is selected from a spherical polymer.

The present disclosure also provides a preparation method of the spherical active carbon, comprising the following steps:
1) carbonizing the spherical polymer;
2) activating the product obtained in step 1).

According to the present disclosure, in step 1), the polymer can be prepared by a polymerization reaction by mixing a monomer and an initiator. As an exemplary example, the polymer can be a homopolymer or a copolymer. Wherein, the homopolymer refers to a polymer prepared by polymerization of a kind of monomer, and the copolymer refers to a polymer prepared by polymerization of two or more kinds of monomers.

According to the present disclosure, the monomer can be selected from the compound having 2 to 60 carbon atoms and at least one carbon-carbon double bond, such as the compound having 2 to 20 carbon atoms and at least one carbon-carbon double bond. For example, the monomer can be selected from the group consisting of ethylene, propylene, isopropylene, butene, isobutylene, pentene, isopentene, neoprene, hexene, isohexene, neohexene, styrene, methylstyrene, acrylic acid, methacrylic acid, methyl acrylate, methyl methacrylate, butadiene, pentadiene, isoprene, Isohexadiene, divinylbenzene, diethylene glycol divinyl ether.

As an alternative, the polymer parent of the copolymer comprises a structural unit derived from a first monomer and a structural unit derived from a second monomer, wherein the first monomer has 2 to 10 carbon atoms and at least one carbon-carbon double bond, and the second monomer has 4 to 15 carbon atoms and at least two carbon-carbon double bonds.

Preferably, in the polymer parent of the copolymer, the structural unit derived from the first monomer accounts for 75% to 98%, preferably 80% to 90% of the total structural unit of the polymer network; and the structural units derived from the second monomer account for 25% to 2%, preferably 20% to 10% of the total structural units of the polymer network.

According to the present disclosure, the first monomer can be selected from one or more from the group consisting of styrene, methylstyrene, acrylic acid, methacrylic acid, methyl acrylate, methyl methacrylate, and a mono olefin with 2-6 carbon atoms. For example, the mono olefin with 2-6 carbon atoms can be selected from ethylene, propylene, isopropylene, butane, isobutylene, pentene, isopentene, neopentene, hexane, isohexene or neohexene.

According to the present disclosure, the second monomer can be selected from one or more from the group consisting of butadiene, prene, isoprene, isohexadiene, divinylbenzene, diethylene glycol divinyl ether.

According to the present disclosure, the polymerization reaction can be a suspension polymerization reaction. Preferably, the polymerization reaction can be also carried out in the presence of water, dispersant or co-dispersant.

For example, the weight ratio of water, dispersant and dispersant is 800-1000 : 0.5-3.0 : 0.05-0.2;

Where the polymer is a homopolymer, the weight ratio of the monomer and the initiator can be 1: 0.003∼0.01.

If present, the weight ratio of the first monomer, the second monomer and the initiator can be 0.75∼0.98 : 0.02∼0.25 : 0.003∼0.01.

Preferably, the water, dispersant and co-dispersant can form an aqueous phase, and the monomer of the homopolymer, or the first monomer, the second monomer and/or the initiator of the copolymer can form an oil phase. Furthermore, the weight ratio of the oil phase to the water phase can be 1:4-6.

According to the present disclosure, the suspension polymerization reactions can comprise the following steps:
the components are added into the reaction kettle, compressed air or nitrogen is introduced into the reaction kettle, the pressure in the reaction kettle is kept at a positive pressure of less than or equal to 0.5 MPa, the temperature is raised to 70-90 °C, hold for 2 h to 24 h, and then the temperature is raised to 100-150 °C, hold for another 4 h to 36 h, then the reaction mixture is washed, dried and sieved to obtain the spherical polymers.

According to a preferred embodiment of the present disclosure, the dispersant can be selected from an inorganic dispersant or an organic dispersant or a combination thereof. For example, the inorganic dispersant is selected from silicate, carbonate or phosphate, or a combination thereof; the organic dispersant is polyvinyl alcohol, gelatin, carboxymethyl cellulose or polyacrylate, or a combination thereof.

According to a preferred embodiment of the present disclosure, the co-dispersant can be selected from sodium dodecyl sulfate, calcium dodecyl benzenesulfonate, sodium dodecyl benzenesulfonate, calcium petroleum sulfonate, sodium petroleum sulfonates, barium stearate, or a combination thereof.

According to a preferred embodiment of the present disclosure, the initiator can be selected from organic peroxide, inorganic peroxide, azo compound, or combinations thereof.

According to a preferred embodiment of the present disclosure, the initiator can be selected from diacyl peroxide compound, dialkyl peroxide compounds, peroxide compound, azodiisobutyronitrile, persulfate, or a combination thereof.

According to a preferred embodiment of the present disclosure, the polymerization reaction can also be carried out in the presence of a pore-foaming agent. The pore-foaming agent can be selected from paraffin, magnesium sulfate, sodium carbonate, gelatin, glycerol, or a combination thereof.

According to the present disclosure, the median particle size D₅₀ of the spherical polymer can range from 0.2-1.5 mm, such as 0.5∼1.3 mm or 0.7∼1.0 mm, particularly 0.5 mm, 0.6 mm, 0.7 mm, 0.8 mm, 0.9 mm, 1.0 mm, 1.1 mm or 1.2 mm.

According to the present disclosure, the polymer can be a sulfonated polymer or a non-sulfonated polymer. Where a non-sulfonated polymers is used, sulfonation can be carried out prior to the carbonization step and/or in situ during carbonization.

As an example, the non-sulfonated polymer can also be prepared according to a known method or commercially available.

The sulfonation can be carried out using raw materials known in the art, such as contacting a non-sulfonated polymer with a sulfonating agent. The sulfonating agent can be one or more selected from sulfuric acid (such as concentrated sulfuric acid), fuming sulfuric acid and SO₃.

According to the present disclosure, the total weight ratio of the non-sulfonated spherical polymer to the sulfonating agent can range from 3:1∼1:3, such as 2:1∼1:2 or 1:1∼1:1.5.

The temperature of the sulfonation step can vary over a wide range. For example, when the sulfonation is performed before the carbonization step, the temperature of the sulfonation step can range from 60-200 °C, such as 70-180 °C or 80-150 °C.

Preferably, the sulfonation reaction can occur within the above temperature range while the temperature being raised. The heating rate can be no more than 10 °C /min, for example no more than 5 °C /min or no more than 3 °C /min.

The time of the sulfonation step can range from 0.5-12 hours, preferably 1-10 hours, for example 2-10 hours.

Preferably, the sulfonation step is carried out in an inert gas atmosphere, wherein the inert gas can be one or more selected from nitrogen, helium and argon.

According to the present disclosure, the carbonization of step 1) can be carried out in an inert atmosphere or in a mixed atmosphere of an inert gas and oxygen.

Generally, the carbonization temperature can be 100-950 °C, such as 150-900 °C or 300-850°C.

When sulfonation is carried out before the carbonization step, the starting temperature of the carbonization step can be equal to or higher than the end temperature of the sulfonation temperature.

Preferably, the carbonization step may react while the temperature being raised within the above temperature range. The heating rate can be no more than 10 °C /min, for example no more than 5 °C /min or no more than 3 °C /min.

Preferably, the carbonization can be carried out sequentially in two or more temperature regions, such as two to ten temperature regions. Preferably, the temperatures of the temperature regions are different from each other. Alternatively, carbonization can be carried out at a gradient rising temperature.

Preferably, the carbonization step can be carried out at the same or different heating rates, with the same or different holding times in different temperature regions.

Preferably, when the carbonization step is carried out sequentially in two or more temperature regions, it can comprise a carbonization in the first temperature region, and then another carbonization in the next temperature region in turn, for example, the second temperature region. For example, the temperature in the first temperature region can be 100-500 °C, such as 150-450 °C. And the temperature in the second temperature region can be higher than that in the first temperature region, such as 500-950 °C or 650-950 °C.

Preferably, the carbonization time is 30 minutes to 10 hours, such as 1 to 8 hours, or 2 to 6 hours.

Preferably, the inert gas is at least one selected from nitrogen, helium, argon.

Preferably, when carbonization is carried out in a mixed atmosphere of inert gas and oxygen, the volume percentage of oxygen in the mixed atmosphere is 1-5%.

It should be understood that if the temperature at which the spherical polymer resides is suitable for sulfonation, the spherical polymer can also be sulfonated in situ during the carbonization.

According to the present disclosure, the activation of step 2) can include a first activation step and a second activation step.

Preferably, the first activation step is carried out in an atmosphere containing water vapor, and the second activation step is carried out in an atmosphere containing CO₂.

Preferably, the temperature of the first activation step is 700-1300 °C, such as 800-1200 °C or 850-950 °C, and the time of the first activation step is 1-24 hours, such as 5-15 hours or 6-12 hours.

Preferably, the atmosphere of the first activation step comprises water vapor, in particular a mixture of water vapor and an inert gas, preferably a mixture of water vapor and nitrogen, or the atmosphere is composed of the above gases.

Preferably, the volume ratio (flow rate ratio) of nitrogen and water vapor is 3:1 or more, such as 4:1∼10:1, preferably 4:1∼8:1.

According to the present disclosure, the atmosphere of the first activation step can comprise no other gases, such as carbon oxides (for example CO₂), oxygen and ammonia.

Preferably, the temperature of the second activation step is 700-1300 °C, such as 800-1200 °C or 850-950 °C, and the time of the second activation treatment is 1-10 hours, such as 3-8 hours.

Preferably, the atmosphere of the second activation step comprises CO₂, such as CO₂ or a mixture of CO₂ and an inert gas, such as a mixture of CO₂ and nitrogen.

Preferably, when the second activation atmosphere comprises a mixture of nitrogen and CO₂, the volume ratio (flow rate ratio) of nitrogen and CO₂ is 10:1∼1:10, such as 10:1∼2:1 or 8:1∼4:1 or 3:1∼2:1.

According to the present disclosure, the atmosphere of the second activation step can comprise no other gases, such as water vapor.

According to the present disclosure, gradient heating can be used for heating. Alternatively, when the temperature is raised to a certain temperature, it can be hold for 1-240 min, such as 5∼150min, and then further raised.

Preferably, the heating process according to the present disclosure can be continuous or intermittent.

The present disclosure also provides the use of the spherical activated carbon as an adsorbent spherical activated carbon according to the present disclosure can be used to adsorb harmful gases, such as one or more selected from CO, H₂S, HCl, SO₂ and NOx. Alternatively, the spherical activated carbon can be used in food industry for the preparation and/or decolorization of a food.

The present disclosure also provides the use of the above spherical activated carbon for preparing a drug.

The present disclosure also provides an adsorbent comprising the above spherical activated carbon.

The present disclosure also provides a protective clothing comprising the above spherical activated carbon.

### Advantageous effect

The present disclosure provides a high-performance spherical activated carbon, preparation method and use thereof. The inventor surprisingly found that according to the preparation method of the present disclosure, a spherical activated carbon with larger particle size, for example a spherical activated carbon of 0.2-1.5 mm or 0.5-1.2 mm, even a spherical activated carbon of 0.7-1.1 mm can be prepared in a good yield with a low cost. Moreover, the activated carbon has excellent physical or mechanical properties and a significantly reduced cracking rate. Moreover, the activated carbon also has excellent adsorption characteristics and can efficiently adsorb harmful gases such as one or more selected from CO, H₂S, HCl, SO₂, NOx. Alternatively, the spherical activated carbon can be used in food industry for the preparation and/or decolorization of a food.

### DETAILED DESCRIPTION

The present disclosure will be further described in detail below in conjunction with specific examples. The following examples are merely illustrative of the present disclosure and are not to be construed as limiting the scope of the present disclosure. The technology that is implemented based on the above-described contents of the present disclosure is encompassed within the scope of the present disclosure.

### Instruments and apparatuses

The specific surface areas in the following examples were determined by a nitrogen physical adsorber model Belsorp Mini II of MicrotracBEL Corp. The compressive strength was determined by the pressure tester of Shanghai Yihuan Instrument Technology Co., Ltd.

### Example 1

### 1.1 Preparation of spherical polymer matrix

18 liters of water were added into a 50-liter polymerization kettle, heated to 45 °C, to which 10 g magnesium carbonate, 20 g of gelatin and 0.15 g of methylene blue were added respectively under stirring. After the system was stirred evenly, an oil phase consisting of 3 kg methyl styrene, 1 kg dipentene and 20 g benzoyl peroxide were added into the system, and then 1.0 kg of paraffin was added, and the polymerization kettle was closed. Clean compressed air was introduced into the polymerization kettle, so that the gas phase pressure in the kettle was kept at 0.02 MPa. Stirring was turned on, the liquid beads in the kettle were adjusted to an appropriate particle size, the system was heated to 80 °C and hold for 12 hours, followed by being heated to 100 °C and hold for 20 hours. The reaction mixture was filtered, washed, dried and sieved to give 2.35 kg white spherical polymer.

### 1.2 Sulfonation and carbonization

The spherical polymer obtained in step 1.1 was mixed with concentrated sulfuric acid at a mass ratio of 1:1, then the mixture was added to an acid resistant rotary tubular furnace. Under a nitrogen atmosphere, the mixture was subjected to the following heating process at the heating rate of 5 °C/min:
heating to 100 °C, holding for 120 minutes;
heating to 150 °C, holding for 240 minutes;
and subjected to the following heating process at the heating rate of 4 °C /min:
   heating to 300 °C, holding for 120 minutes;
   heating to 500 °C, holding for 240 minutes;
   then heating to 650 °C, holding for 100 minutes.

After cooling, the carbonized product was obtained.

### 1.3 Activation

In a rotary tubular furnace, under the mixed atmosphere of water vapor and nitrogen with a flow rate ratio of 1:4.5 (L/min), the carbonized product obtained in step 1.2 was heated to 800 °C at a rate of 3 °C /min, hold for 360 minutes, then under the mixed atmosphere of CO₂ and nitrogen with a flow rate ratio of 1:4 (L/min), it was further heated to 950 °C at a rate of 3 °C /min, hold for 120 minutes. After cooling, spherical activated carbon GSC1 was obtained, with a yield of 37% based on the polymer, a median particle size of 0.75 mm, a median pore size of 3.50 nm, a specific surface area of 958 m²/g, a compressive strength of 79.90 N, a bulk density of 470 g/L, and a cracking rate of 4.72%.

### Example 2

### 2.1 Preparation of spherical polymer matrix

20 liters of water were added into a 50-liter polymerization kettle, heated to 40 °C, to which 10 g of calcium carbonate, 20g polyvinyl alcohol and 0.15g calcium petroleum sulfonate were added respectively under stirring. After the system was stirred evenly, an oil phase consisting of 3 kg styrene, 1kg isoprene and 20 g azodiisobutyronitrile were added into the system, and then 1.6 kg glycerol was added, and the polymerization kettle was closed. clean compressed air was introduced into the polymerization kettle, so that the gas phase pressure in the kettle was kept at 0.04 MPa. Stirring was turned on, the liquid beads in the kettle were adjusted to an appropriate particle size, the system was heated to 80 °C and hold for 12 hours, followed by being heated to 100 °C and hold for 20 hours. The reaction mixture was filtered, washed, dried and sieved to give 2.76kg white spherical polymer.

### 2.2 Sulfonation and carbonization

The spherical polymer obtained in step 2.1 was mixed with SO₃ at a mass ratio of 1:1.5, then the mixture was added to an acid resistant rotary tubular furnace. Under a helium atmosphere, the mixture was subjected to the following heating process at the heating rate of 4 °C /min:
heating to 80 °C, holding for 60 minutes;
heating to 150 °C, holding for300 minutes;
and subjected to the following heating process at the heating rate of 3 °C /min under a mixed atmosphere containing 5% by volume of oxygen:
   heating to 200 °C, holding for 90 minutes;
   heating to 500 °C, holding for 320 minutes;
   then heating to 600 °C, holding for 120 minutes.

After cooling, the carbonized product was obtained.

### 2.3 Activation

In a rotary tubular furnace, under the mixed atmosphere of water vapor and nitrogen with a flow rate ratio of 1:4, the carbonized product obtained in step 2.2 was heated to 950 °C at a rate of 3 °C /min, hold for 360 minutes, then under the mixed atmosphere of CO₂ and nitrogen with a flow rate ratio of 1:3 (L/min), it was further heated to 950 °C at a rate of 4 °C /min, hold for 120 minutes. After cooling, spherical activated carbon GSC2 was obtained, with a yield of 39% based on the polymer, a median particle size of 1.15 mm, a median pore size of 1.90 nm, a specific surface area of 956 m²/g, a compressive strength of 52.76 N, a bulk density of 460 g/L, a cracking rate of 4.62%.

### Example 3

### 3.1 Preparation of spherical polymer matrix

20 liters of water were added into a 50-liter polymerization kettle, heated to 40 °C, to which 12 g magnesium carbonate, 25g sodium carboxymethylcellulose and 0.18g calcium dodecylbenzene sulfonate were added respectively under stirring. After the system was stirred evenly, an oil phase consisting of 3.6 kg divinylbenzene, 1.2 kg diethylene glycol divinyl ether and 25 g sodium persulfate were added into the system, and then 2.2 kg sodium carbonate was added, the polymerization kettle was closed. Clean compressed air was introduced into the polymerization kettle, so that the gas phase pressure in the kettle was kept at 0.05 MPa. Stirring was turned on, the liquid beads in the kettle were adjusted to an appropriate particle size, the system was heated to 90 °C and hold for 9 hours, followed by being heated to 120 °C and hold for 20 hours. The reaction mixture was filtered, washed, dried and sieved to give 3.12 kg white spherical polymer.

### 3.2 Sulfonation and carbonization

The spherical polymer obtained in step 3.1 was added into the polymerization kettle, to which 10 kg fuming sulfuric acid with mass concentration of 105% were added into the polymerization kettle. The system was heated to 110°C and hold for 16 hours, followed by adding water dropwise slowly after the temperature decreased. 1/3 liquid was suctioned out when the kettle was full, and the water was sequentially added dropwise, so that the sulfuric acid concentration in the kettle was less than 5%. The product was dried to give 4.28 kg spherical polymer. Under a nitrogen atmosphere, the polymer microsphere was subjected to the following heating process at a heating rate of 3 °C/min:
heating to 120 °C, holding for 110 minutes;
heating to 180 °C, holding for 250 minutes;
and subjected to the following heating process at the heating rate of 3 °C /min under a mixed atmosphere containing 1% by volume of oxygen:
   heating to 250 °C, holding for 360 minutes;
   heating to 450 °C, holding for 240 minutes;
   then heating to 700 °C, holding for 90 minutes.

After cooling, carbonization products were obtained.

### 3.3 Activation

In a rotary tubular furnace, under the mixed atmosphere of water vapor and nitrogen with a flow rate ratio of 1:7 (L/min), the carbonized product obtained in step 3.2 was heated to 800 °C at a rate of 3 °C /min, hold for 420 minutes, then under the mixed atmosphere of CO₂ and nitrogen with a flow rate ratio of 1:7 (L/min), it was further heated to 950 °C at a rate of 4 °C/min, hold for 200 minutes. After cooling, spherical activated carbon GSC3 was obtained, with a yield of 42% based on the polymer, a median particle size of 0.90 mm, a median pore size of 2.95 nm, a specific surface area of 1011 m²/g, a compressive strength of 78.24 N, a bulk density of 514 g/L, and a cracking rate of 3.32%.

### Example 4

### 4.1 Preparation of spherical polymer matrix

18 liters of water were added into a 50-liter polymerization kettle, heated to 45 °C, to which 10 g magnesium carbonate, 20g gelatin and 0.15g methylene blue were added respectively under stirring. After the system was stirred evenly, an oil phase consisting of 3 kg methylstyrene, 1 kg dipentene and 20 g benzoyl peroxide were added into the system, and then 1.3 kg magnesium sulphate was added, and the polymerization kettle was closed. Clean compressed air was introduced into the polymerization kettle, so that the gas phase pressure in the kettle was kept at 0.02 MPa. Stirring was turned on, the liquid beads in the kettle were adjusted to an appropriate particle size, the system was heated to 80 °C and hold for 12 hours, followed by being heated to 100 °C and hold for 20 hours. The reaction mixture was filtered, washed, dried and sieved to give 2.51kg white spherical polymer.

### 4.2 Sulfonation and carbonization

The spherical polymer obtained in step 4.1 was mixed with concentrated sulfuric acid at a mass ratio of 1:1.3, then the mixture was added to an acid resistant rotary tubular furnace. Under a nitrogen atmosphere, the mixture was subjected to the following heating process at the heating rate of 2 °C/min:
heating to 60 °C, holding for 60 minutes;
heating to 130 °C, holding for 100 minutes;
heating to 160 °C, holding for 150 minutes;
and subjected to the following heating process at the heating rate of 3 °C/min under a mixed atmosphere containing 3% by volume of oxygen:
   heating to 400 °C, holding for 240 minutes;
   heating to 550 °C, holding for 240 minutes;
   then heating to 700 °C, holding for 100 minutes.

After cooling, the carbonized product was obtained.

### 4.3 Activation

In a rotary tubular furnace, under the mixed atmosphere of water vapor and nitrogen with a flow rate ratio of 1:6.5 (L/min), the carbonized product obtained in step 4.2 was heated to 750 °C at a rate of 3 °C /min, hold for 300 minutes, then under the mixed atmosphere of CO₂ and nitrogen with a flow rate ratio of 1:5.5 (L/min), it was further heated to 980 °C at a rate of 2 °C /min, hold for 300 minutes. After cooling, spherical activated carbon GSC4 was obtained, with a yield of 41% base on polymer, a median particle size of 0.55 mm, a median pore size of 2.19 nm, a specific surface area of 704 m²/g, a compressive strength of 124.72 N, a bulk density of 675 g/L, and a cracking rate of 4.92%.

### Example 5 Determination of iodine adsorption value

The iodine adsorption values of the spherical activated carbons GSC1 to GSC4 prepared in the above Example 1-4 were determined according to GB/T 12496.8-2015, named "TEST METHOD OF WOODEN ACTIVATED CARBON: DETERMINATION OF IODINE ADSORPTION VALUE". The results were shown in Table 1.

**Table 1 iodine adsorption values of the spherical activated carbons prepared in Example 1-4**

| Example | Iodine adsorption value (mg/g) |
|---|---|
| Example 1 | 800 |
| Example 2 | 780 |
| Example 3 | 950 |
| Example 4 | 600 |

The above content explained the embodiment of the disclosure. However, the present disclosure is not limited to the above embodiments. Any modification, equivalent replacement, improvement, etc. made within the spirit and principle of the invention shall be included in the protection scope of the disclosure.

## Claims

1. A spherical activated carbon, wherein the specific surface area B of the activated carbon is less than 1250 m²/g;
preferably, 600 m²/g≤B≤1200 m²/g; for example, 700 m²/g≤B≤1100 m²/g.

2. The spherical activated carbon according to claim 1, wherein the median particle size D₅₀ of the spherical activated carbon can be 0.2-1.5mm, such as 0.5-1.3 mm, for example 0.7-1.2 mm;
preferably, the median pore size of the spherical activated carbon can be 1∼4 nm, such as 1.5∼3.8 nm, for example 1.8∼3.8 nm;
preferably, the compressive strength of the spherical activated carbon can be 10∼300N, such as 40-150N, for example 50-140N, for example 50-130 N;
preferably, the cracking rate of the spherical activated carbon can be less than 10.0%, such as 1.0∼10.0%, for example 1.5∼6.0%, preferably less than 5.0%, for example 3.0∼5.0%.

3. The spherical activated carbon according to claim 1 or 2, wherein the iodine adsorption value of the spherical activated carbon is 400-1100 mg/g, preferably 500-1000 mg/g, for example 600-950 mg/g;
preferably, the bulk density of the spherical activated carbon can be 300-1000 g/L, preferably 400-800 g/L, for example 450-700 g/L.

4. The spherical activated carbon according to any one of claims 1 to 3, wherein the raw material for preparing the spherical activated carbon is a spherical polymer.

5. A preparation method of the spherical activated carbon according to any one of claims 1 to 4, comprising the following steps:
1) carbonizing the spherical polymer;
2) activating the product obtained in step 1).

6. The preparation method according to claim 5, wherein the carbonization temperature of step 1) can be 100-950 C, for example 150-900 °C, such as 300-850°C;
the carbonization time is 30 minutes to 10 hours, for example 1 to 8 hours, such as 2 to 6 hours;
the activation of step 2) can comprise a first activation step and a second activation step; the temperature of the first activation step is 700-1300 °C, for example 800-1200 °C, such as 850-950 °C;
the time of the first activation step is 1-24 hours, for example 5-15 hours, such as 6-12 hours preferably, the atmosphere of the first activation step comprises water vapor, in particular a mixture of water vapor and an inert gas, preferably a mixture of water vapor and nitrogen, or the atmosphere is composed of the above gases;
preferably, the volume ratio (flow rate ratio) of nitrogen and water vapor is 3:1 or more, for example 4:1∼10:1, preferably 4:1∼8:1;
the temperature of the second activation step is 700-1300 °C, preferably 800-1200 °C, for example 850-950 °C;
the time of the second activation step is 1-10 hours, for example 3-8 hours;
preferably, the atmosphere of the second activation step comprises CO₂, for example CO₂ or a mixture of CO₂ and an inert gas, for example a mixture of CO₂ and nitrogen;
preferably, where the second activation atmosphere comprises a mixture of nitrogen and CO₂, the volume ratio (flow rate ratio) of nitrogen and CO₂ can be 10:1∼1:10, for example 10:1∼2:1, such as 8:1∼4:1, for example 3:1∼2:1.

7. Use of the spherical activated carbon according to any one of claims 1 to 4 as an adsorbent; preferably, the spherical carbon is used to adsorb harmful gases, for example one or more selected from the group consisting of CO, H₂S, HCl, SO₂ and NOx;
preferably, the spherical activated carbon is used in food industry for the preparation and/or decolorization of a food.

8. Use of the spherical activated carbon according to any one of claims 1 to 4 in the preparation of drugs.

9. An adsorbent, comprising the spherical activated carbon according to any one of claims 1 to 4.

10. A protective clothing, comprising the above spherical activated carbon according to any one of claims 1 to 4.
